# FASCICULE DE BREVET EUROPEEN

(11) **EP 3 478 368 B1**
(45) Date de publication et mention de la délivrance du brevet: **07.06.2023**
(21) Numéro de dépôt: 16774531.4
(22) Date de dépôt: 12.09.2016
(51) Int. Cl.: A61Q 19/08, A61K 8/9789, A61K 8/34, A61K 36/15, A61K 36/87, A61K 31/09, A61K 31/341, A61K 31/355, A61K 31/365, A61K 31/366, A61P 39/06, A61K 31/05

(54) **COMPOSITION POUR LUTTER CONTRE LES SIGNES DU VIEILLISSEMENT DE LA PEAU ET DES PHANERES**
ZUSAMMENSETZUNG ZUR BEKÄMPFUNG DER ANZEICHEN DER ALTERUNG DER HAUT UND HAARE UND NÄGEL
COMPOSITION FOR COMBATING THE SIGNS OF AGEING OF THE SKIN AND HAIR AND NAILS

(30) Priorité: 01.07.2016 FR 1656318
(43) Date de publication de la demande: 08.05.2019
(73) Titulaire: Tomcat International, London, W1W 8DQ (GB)
(72) Inventeur: THOMAS, Mathilde, 75008 Paris (FR); THOMAS, Bertrand, 75008 Paris (FR)
(74) Mandataire: Germain Maureau
(86) Numéro de dépôt international: PCT/FR2016/052294
(87) Numéro de publication internationale: WO 2018/002453

(56) Documents cités:
- EP-A1- 0 698 595
- FR-A1- 3 017 289
- JP-A- 2003 160 433
- US-A1- 2010 047 294
- DATABASE GNPD [Online] MINTEL; 1 février 2016 (2016-02-01), Caudalie: "Eye Lifting Balm", XP002764744, Database accession no. 3658627
- DATABASE GNPD [Online] MINTEL; 1 décembre 2015 (2015-12-01), CVS Pharmacy: "Accelerated Wrinkle Repair Moisturizer", XP002764745, Database accession no. 3677493
- BJARNE HOLMBOM ET AL: "Knots in trees ? A new rich source of lignans", PHYTOCHEMISTRY REVIEWS, KLUWER, NL, vol. 2, no. 3, 1 octobre 2003 (2003-10-01) , pages 331-340, XP008104241, ISSN: 1568-7767, DOI: 10.1023/B:PHYT.0000045493.95074.A8 [extrait le 2004-11-08]

## Description

La présente invention concerne des compositions cosmétiques et des associations d'actifs améliorant l'aspect de la peau ou des phanères et notamment utiles comme agent pour lutter contre certains signes du vieillissement de la peau ou des phanères et/ou comme agent anti-pollution.

L'invention porte en particulier sur des associations renforçant les défenses intrinsèques de la peau contre les agents oxydants. Elle porte également sur leur utilisation cosmétique pour lutter contre les signes du vieillissement de la peau ou des phanères, pour améliorer l'éclat du teint ou pour diminuer les cernes. Elle porte également sur un procédé de traitement cosmétique comprenant l'application topique de l'association ou d'une composition la contenant.

La peau est constituée de deux compartiments, l'un superficiel, l'épiderme, et un plus profond, le derme, qui interagissent. L'épiderme humain est composé principalement de trois types de cellules, qui sont les kératinocytes, très majoritaires, les mélanocytes et cellules de Langerhans. Chacun de ces types cellulaires contribue par ses fonctions propres au rôle de la peau dans l'organisme, et notamment à la protection contre les agressions extérieures.

L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin la couche cornée (ou stratum corneum), constituée d'un ensemble de couches de kératinocytes au stade terminal de leur différenciation appelés cornéocytes.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.

La cohésion entre l'épiderme et le derme est assurée par la jonction dermo-épidermique.

L'oxydation cellulaire et l'accumulation de radicaux libres jouent un rôle important dans les processus de vieillissement.

Les sources de radicaux libres peuvent être endogènes, résultat de processus métaboliques normaux ou de la respiration ; ils peuvent être aussi augmentés par des facteurs environnementaux comme l'exposition à la pollution, à la fumée ou aux UV. L'action de ces radicaux libres sur la peau peut entrainer un stress oxydatif.

Ces phénomènes de vieillissement qu'ils soient d'origine intrinsèques ou extrinsèques, sont des phénomènes physiologiques naturels. Mais il existe toujours un besoin chez un grand nombre d'hommes et de femmes d'améliorer l'apparence de leur peau ou de leurs phanères et de diminuer ou retarder les signes liés au vieillissement.

La demande JP2003160433 décrit des compositions complexes présentant une activité anti-MMP (matrix metallo proteinase) contenant un mélange de *Theobroma* cacao avec différents extraits végétaux.

Les cellules, en particulier les cellules cutanées, ont développé des mécanismes de défense contre ces agents oxydants et le stress oxydatif, et pour éviter ses effets indésirables qui contribuent au vieillissement.

Il s'agit d'une part du glutathion, sous sa forme réduite ; d'autre part, de nombreuses enzymes ont une activité antioxydante, telles que les superoxyde dismutases (SOD), les catalases, ou les hème-oxygénases.

L'utilisation d'agents antioxydants a déjà été proposée dans le domaine cosmétique, notamment en application topique. On connait par exemple le tocophérol (vitamine E) ou ses dérivés, la vitamine C ou ses dérivés, les caroténoïdes, l'ubiquinone ou le thé vert.

La demande FR 3 017 289 décrit des compositions à fort pouvoir antioxydant direct contenant des dérivés de polyphénols et de la vitamine C.

Toutefois, il existe toujours un besoin de disposer de nouveaux actifs susceptibles d'exercer une action cosmétique antioxydante globale et notamment de lutter contre le stress oxydatif de la peau et/ou des cheveux et ses effets.

Il a maintenant été trouvé dans le cadre de la présente invention que des extraits de la plante *Picea abies* qui ont une activité antioxydante directe sont aussi capables de favoriser les deux voies de défense endogènes des cellules contre le stress oxydatif.

En outre l'effet antioxydant sur les défenses cellulaires se trouve augmenté de façon synergique lorsque ces extraits sont associés à des polyphénols du type de ceux présents dans la vigne ; ces polyphénols peuvent être sous forme native ou stabilisée.

C'est pourquoi la présente invention concerne une association de
(a) au moins un polyphénol extrait de la vigne choisi parmi les monomères et oligomères de proanthocyanidines (OPC), les monomères et oligomères de flavan-3-ols, leurs dérivés et leurs mélanges, les dérivés de polyphénols étant des esters de polyphénols, et
(b) un extrait d'épicéa comprenant au moins un lignane choisi parmi : hydroxymatairésinol, sécoisolaricirésinol, conidendrine, laricirésinol et liovile
et a pour objet des compositions, en particulier des compositions pour application topique telles que des compositions cosmétiques, contenant au moins ladite association.

L'invention a encore pour objet les associations telles que définies dans la présente demande, ou compositions les contenant, pour leur utilisation pour prévenir ou diminuer les désordres cutanés liés au stress oxydatif, et/ou pour stimuler les défenses des cellules cutanées contre le stress oxydatif.

L'invention a également pour objet l'utilisation cosmétique -non thérapeutique- d'une association de (a) au moins un polyphénol extrait de la vigne choisi parmi les monomères et oligomères de proanthocyanidines (OPC), les monomères et oligomères de flavan-3-ols, leurs dérivés et leurs mélanges, les dérivés de polyphénols étant des esters de polyphénols, et (b) un extrait d'épicéa comprenant au moins un lignane choisi parmi hydroxymatairésinol, sécoisolaricirésinol, conidendrine, laricirésinol et liovile, ou d'une composition cosmétique la contenant pour lutter contre les signes du vieillissement de la peau ou des phanères et/ou contre les signes cutanés liés à l'environnement et notamment à la pollution.

Des extraits de *Picea abies* (ou épicéa) utiles selon l'invention ont été décrits dans la demande WO 2009/101261, pour leur activité antibactérienne et comme agent de photoprotection dans des produits anti-UV. L'activité de ces extraits est basée principalement sur une réaction d'oxydo-réduction permettant de neutraliser les radicaux libres ou par piégeage de ces radicaux.

De manière inattendue, il a maintenant été trouvé que des extraits de *Picea abies,* ou épicéa, augmentent la production de GSH par les cellules cutanées et améliorent ainsi leur résistance et celle de la peau au stress oxydatif.

Le glutathion est un antioxydant présent dans les cellules à l'état basal, principalement sous forme réduite, le GSH. Une petite fraction est sous forme oxydée, le GSSG. Les extraits d'épicéa selon l'invention sont capables d'augmenter la synthèse de GSH d'au moins 10% par rapport à des cellules cutanées n'ayant pas été en contact avec ledit extrait ; cette augmentation peut être d'au moins 20 %, voire d'au moins 30%, et peut être supérieure ou égale à 60%, et notamment égale à environ 66%.

En outre, les extraits d'épicéa activent, à des doses non toxiques, la synthèse cellulaire de l'enzyme hème-oxygénase 1 ou HO-1.

L'hème-oxygénase est une enzyme intervenant dans la dégradation de l'hémoglobine. L'isoforme 1 de l'hème oxygénase (HO-1) est une enzyme de phase II présente dans la peau humaine normale ; elle joue un rôle dans le système physiologique de détoxification de la peau et de protection anti oxydante, en réponse à des stimuli comme le stress oxydatif.

Un extrait d'épicéa est donc utile pour favoriser les défenses endogènes de la peau contre les espèces oxydantes, par deux voies complémentaires.

Il a par ailleurs été découvert dans le cadre de la présente invention que des polyphénols, en particulier des polyphénols extraits de vigne, ou leurs dérivés ont aussi un effet de stimulation de la synthèse de l'enzyme HO-1.

De manière inattendue, une association d'extrait d'épicéa et de polyphénols extraits de vigne ou de dérivés de polyphénols extraits de la vigne a un effet synergique sur l'augmentation de la production (synthèse) de HO-1 par les cellules, en particulier les cellules de la peau.

L'association d'un extrait d'épicéa et de polyphénols extraits de vigne ou d'esters de tels polyphénols aura donc une activité améliorée en stimulant les défenses endogènes des cellules cutanées contre les espèces oxydantes à deux niveaux complémentaires, par la voie du glutathion (augmentation de synthèse de GSH) et la voie enzymatique de la détoxification (augmentation de la synthèse d'HO-1).

Les polluants atmosphériques tels que les matières particulaires ou les polluants gazeux comme le dioxyde de soufre, l'ozone et les oxydes d'azote, la fumée de cigarette ont une activité d'initiateurs de radicaux libres qui sont la source de phénomènes d'oxydation provoquant chez les êtres vivants des dommages cellulaires. Les cellules d'organes qui sont en contact direct et permanent avec le milieu extérieur, comme la peau, le cuir chevelu et certaines muqueuses, sont particulièrement sensibles à ces effets des polluants, qui se traduisent notamment par un vieillissement accéléré de la peau, avec un teint manquant d'éclat, des taches pigmentaires et une formation précoce de rides et de ridules, et aussi par une diminution de la vigueur et un aspect terne des cheveux.

L'invention concerne donc l'utilisation de l'association (ou les compositions la contenant), pour lutter contre les signes du vieillissement de la peau ou des phanères, qu'il soit intrinsèque ou extrinsèque, et pour lutter contre les signes cutanés liés à l'effet de l'environnement et de la pollution.

Outre les rides et les ridules, l'apparition de marques pigmentaires, la diminution de l'épaisseur de la peau, sa perte d'élasticité et son affaissement sont également des changements observés au cours du vieillissement. Outre son vieillissement continu, la peau évolue en permanence en fonction de l'état de fatigue de la personne, qui se traduit par des signes tels que poches et cernes sous les yeux, traits tirés et teint terne.

L'association, ou une composition la contenant, sera notamment utile pour diminuer ou prévenir les désordres cutanés tels qu'un aspect terne du teint, l'hyperpigmentation de la peau ou l'hétérogénéité de sa pigmentation et les taches pigmentaires, les rides et les ridules, l'amincissement de la peau et la perte de fermeté et/ou d'élasticité de la peau.

Par peau, on entend la peau du visage et/ou du corps, le cuir chevelu et les muqueuses ou semi-muqueuses (comme par exemple les lèvres). Par phanères, on entend les cheveux, les poils, les cils, les ongles, et de préférence les cheveux.

Par ailleurs la présence de cernes sous les yeux est un signe qui contribue à une apparence de fatigue ou de vieillissement, et il est souhaitable de les diminuer. Parmi les causes à l'origine de leur présence, on peut citer une accumulation d'hémoglobine dans la zone fine et très vascularisée située sous l'œil. La stimulation de la dégradation de l'hémoglobine dans cette région, contribue à la disparition des zones sombres et donc à l'atténuation ou la disparition des cernes.

L'invention a donc également pour objet l'utilisation de l'association d'un extrait d'épicéa et de polyphénols selon l'invention, ou les compositions les contenant, pour améliorer l'éclat de la peau et du teint et/ou prévenir ou diminuer l'apparition de cernes autour et/ou sous les yeux.

Dans la présente description, pour les intervalles exprimés "entre... et..." ou "de... à... ", il est entendu que les bornes sont incluses.

L'épicéa ou épicéa commun (*Picea abies*) est une espèce d'arbres résineux de la famille des Pinacées et du genre Picea. Les extraits adaptés à la mise en oeuvre de l'invention sont de préférence obtenus à partir du bois et de l'écorce de l'arbre, en particulier du bois présent au niveau des noeuds du bois ou à leur voisinage.

Les extraits d'épicéa pour la mise en oeuvre de l'invention contiennent de préférence au moins 5%, et notamment entre 5 et 15% de lignanes par rapport au poids de l'extrait (exprimé en matière sèche), avec au moins un lignane choisi parmi : hydroxymatairésinol, sécoisolaricirésinol, conidendrine, laricirésinol et liovile. Un extrait convenant particulièrement à la mise en oeuvre de l'invention contient au moins 4%, notamment entre 4 et 10% et en particulier environ 5 à 6% d' hydroxymatairésinol. La teneur de chacun des autres lignanes dans l'extrait est généralement comprise entre 0,01 et 5%, et notamment d'environ 0,1 à 2%. On utilise notamment un extrait (titré à 8 % en lignanes) solubilisé dans la glycérine.

Les polyphénols adaptés à la mise en oeuvre de l'invention sont choisis parmi les monomères et oligomères de proanthocyanidines (OPC), les monomères et oligomères de flavan-3-ols. De tels polyphénols sont extraits de la vigne. Ils peuvent être obtenus à partir de différentes parties de la plante *Vitis vinifera,* par exemple des fruits ou de parties du fruit comme les pépins ou la peau, mais aussi à partir des sarments de vigne.

D'autres polyphénols convenant à l'invention sont les monomères et oligomères de flavan-3-ols, en particulier catéchine, épicatéchine, gallocatéchines, épigallocatéchines (par exemple épigallocatéchine gallate) et oligomères de proanthocyanidine ou OPC, les anthocyanes ou les flavanones ; on peut citer encore les flavonols, dihydroflavonols, flavones et isoflavones.

Avantageusement, les polyphénols ou les dérivés qui sont des esters de polyphénols comprennent du resvératrol et/ou des catéchines, et/ou les oligomères de catéchines ou de resvératrol, ou des dérivés de ces composés.

Les oligomères de catéchines comprennent de préférence de 2 à 6 unités catéchiques (catéchine ou épicatéchine) et sont aussi appelés OPC.

Ces polyphénols peuvent être sous forme native ou stabilisée.

Par dérivés des polyphénols on entend des molécules stabilisées dans lesquelles tout ou partie des groupements hydroxyles sont estérifiés. Selon un mode de réalisation préféré, l'estérification est formée avec un acide gras saturé ou insaturé. L'acide gras peut notamment être choisi parmi l'acide butyrique, valérique, hexanique, sorbique, laurique, palmitique, stéarique, oléique, linoléique, linolénique, alpha-linolénique, arachidonique, écosapentaénoïque et docosahexaénoïque.

Les unités monomères des flavon-3-ols répondent à la structure (I) suivante 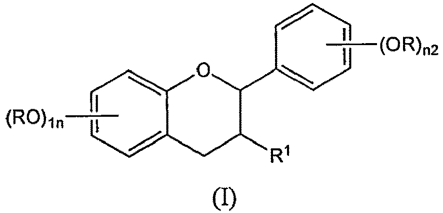 dans laquelle - R1 représente un groupe -OR2, un atome d'hydrogène, ou un substituant R3 ; R2 et R3 identiques ou différents, étant tels que défini ci-dessous pour R4
- au moins la majorité des substituants R représentent un groupe -COR4, R4 étant un radical alkyle d'au moins deux atomes de carbone, linéaire ou ramifié, saturé ou insaturé, un radical aryle, arylalkyle ou arylalkylène,
- le ou les autres substituants R qui ne représentent pas un groupe -COR4 étant un atome d'hydrogène, ou un groupe alkyle, et
- n1 et n2, identiques ou différents l'un de l'autre, sont des nombres de 1 à 3, correspondant au nombre de substitutions sur un cycle,
les motifs monomères étant reliés dans les oligomères et les polymères par des liaisons carbone-carbone et /ou un pont éther entre les unités.

Dans les oligomères et polymères de l'invention, les liaisons entre les atomes de carbone des motifs successifs sont situées entre le C-4 d'un motif et le C-6 ou le C-8 d'un autre motif.

Dans d'autres oligomères et polymères, au moins 2 motifs sont reliés en outre par un pont oxygène.

Dans une composition correspondante, les oligomères ou les polymères de polyphénols répondent à la formule 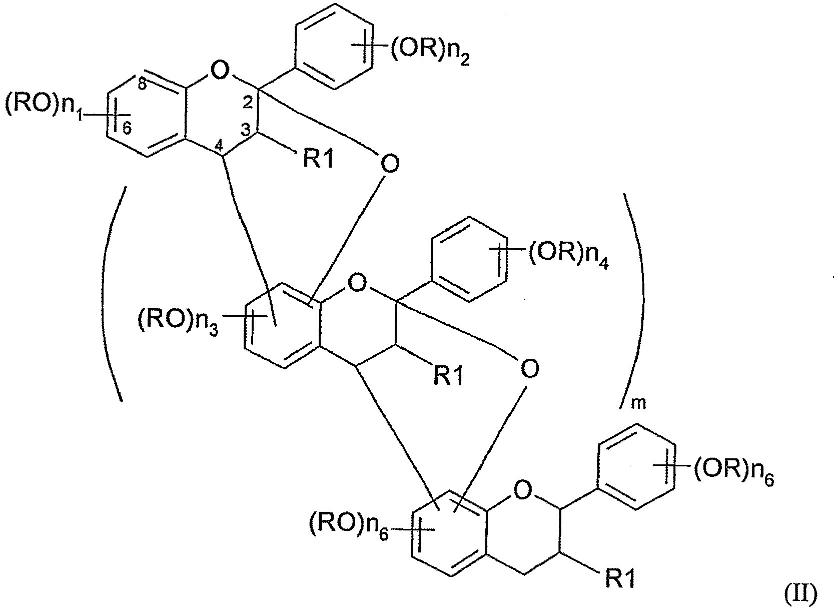 dans laquelle R1 et R sont tels que définis ci-dessus, n1 à n6, identiques ou différents, sont des nombres de 1 à 3 et m est un nombre de 0 à 100, plus particulièrement de 1 à 10.

Dans la formule (II), m est de préférence un nombre de 11 à 100 ou, dans une variante, m est égal à 0.

L'invention vise en particulier une composition caractérisée en ce que R représente un radical d'acide gras saturé ou insaturé choisi plus spécialement dans le groupe comprenant les radicaux de l'acide butyrique, valérique, hexanique, sorbique, laurique, palmitique, stéarique, oléique, linoléique, linolénique, α-linolénique, arachidonique, écosapentaénoïque et docosahexaénoïque.

Dans encore d'autres polyphénols de formule (II), R2 représente un groupe phényle ou arylalkyle ou arylalkylène, le radical alkyle ou alkylène étant plus particulièrement en C1 à C8, notamment en C1 à C4. A titre d'exemple de groupes arylalkyle ou arylalkylène, on citera le groupe benzyle et styryle.

L'invention vise en particulier les dérivés de flavanols, notamment ceux dans lesquels R1 est un groupe -0R2, R2 étant tel que défini ci-dessus. Il s'agit de manière préférée d'esters de dérivés de flavanols appartenant à la série catéchique.

Dans ces esters, les groupes oxygénés sont généralement au nombre de 5 par motif flavanolique, et occupent les positions 3, 5, 7, 3' et 4'.

Dans une composition préférée, les polyphénols sont des extraits flavonoïdiques de sources végétales, et précisément de la vigne et plus spécialement des pépins de raisins (OPC), renfermant des monomères, des oligomères et/ou des polymères d'unités répondant à la formule (I).

L'invention vise également une composition caractérisée en ce que les polyphénols correspondent aux diastéréoisomères et/ou aux régioisomères desdits polyphénols flavonoïdiques.

Selon un mode de réalisation particulier, l'ensemble des groupements hydroxyles du polyphénol sont estérifiées, le dérivé de polyphénol ne présente donc plus de fonction OH libre.

De tels esters peuvent notamment être préparés par le procédé décrit dans la demande WO 2011/128714.

On utilise en particulier des polyphénols extraits de sarments de vigne, notamment le resvératrol, et leurs dérivés. Des polyphénols particulièrement adaptés à la mise en oeuvre de l'invention sont les catéchines, oligomères de catéchines ou OPC, tels que ceux extraits de pépins de raisin, et les dérivés de ces polyphénols, en particulier leurs esters.

Il est entendu que dans le cadre de l'invention, les mélanges en toutes proportions des différents polyphénols et de leurs dérivés estérifiés en partie ou en totalité, tels que définis dans ce qui précède peuvent être utilisés.

Avantageusement, dans une composition selon l'invention, les polyphénols mentionnés en (a) sont extraits de la vigne, et comprennent des OPC extraits de pépin de raisin ; en particulier l'association ou la composition comprend des dérivés de polyphénols qui sont des esters de polyphénols.

Selon un mode de réalisation particulier, les polyphénols sont présents dans l'association ou la composition sous forme d'extraits de pépins de raisin, éventuellement stabilisés pour obtenir des dérivés de polyphénols dans lesdits extraits.

De tels polyphénols comprennent généralement au moins 20% (en poids, par rapport au poids de polyphénols totaux) de monomères et/ou dimères de catéchines; par exemple, la teneur en dimères de proanthocyanidine est supérieure ou égale à 20 % (en poids, par rapport au poids de polyphénols totaux), et/ou la teneur en [catéchine et épicatéchine] est supérieure ou égale à 20 % (en poids, par rapport au poids de polyphénols totaux).

Selon un mode de réalisation de l'invention, les extraits de pépin de raisin sont essentiellement dépourvus de composants autres que des polyphénols.

Une composition, en particulier une composition cosmétique selon l'invention peut notamment comprendre des polyphénols ou les dérivés de polyphénols mentionnés en (a) dans une concentration de 0,01 à 10% en poids par rapport au poids total de la composition, et notamment supérieure ou égale à 0,05%. Selon un mode de réalisation de l'invention, dans la composition les polyphénols ou les dérivés de polyphénols mentionnés en a) sont présents par exemple à une concentration de 0,1 à 5 % ; en particulier la composition contient de 0,1 à 5 % de dérivés estérifiés de polyphénols de pépins de raisin.

Une composition en particulier une composition cosmétique selon l'invention peut notamment comprendre l'extrait d'épicéa, en particulier un extrait de bois d'épicéa présent à une concentration de 0,01 à 10% en poids par rapport au poids total de la composition. L'extrait d'épicéa peut par exemple être présent à une concentration supérieure ou égale à 0,05%, et notamment supérieure ou égale à 0,1%. Les extraits de bois d'épicéa tels que définis dans ce qui précède peuvent ainsi être présents à une concentration de 0,1 à 5%, voire de 0,5 à 5% ou de 0,5 à 3% en poids par rapport au poids total de la composition.

Une composition selon l'invention contient par exemple un extrait de pépin de raisin, éventuellement stabilisé par estérification des polyphénols, à une concentration de 0,01 à 10% en poids par rapport au poids total de la composition et un extrait de bois d'épicéa présent à une concentration de 0,01 à 10% en poids par rapport au poids total de la composition, par exemple à une concentration supérieure ou égale à 0,05%.

Avantageusement, dans une composition cosmétique selon l'invention le rapport massique entre les polyphénols, en particulier les polyphénols extraits de vigne (notamment les polyphénols de pépins de raisin, riches en OPC) ou leurs dérivés et l'extrait d'épicéa est de 0,1 à 5 exprimés en polyphénols/ lignanes d'épicéa (ou en polyphénols extraits de vigne/ lignanes d'épicéa) et préférentiellement un rapport polyphénols de raisins/ lignanes d'épicéa compris entre 0,5 et 3. En effet, il a été observé que dans ces ratios l'activité synergique sur les défenses anti-oxydantes endogènes médiée par l'enzyme HO-1 est optimale.

En particulier, sur des fibroblastes de peau humaine, la synthèse de HO-1 est augmentée d'au moins 30%, notamment d'au moins 40% par rapport à l'augmentation qui serait attendue par un simple effet additif de chacun des deux composants de l'association ; cette synergie peut conduire à une augmentation de plus de 70% par rapport à la somme des effets de chacun des composants de l'association.

Les compositions selon l'invention sont de préférence des compositions cosmétiques adaptées à une application sur la peau ou les phanères. La composition selon l'invention contient l'association de polyphénols et d'extraits d'épicéa telle que définie précédemment, et un milieu physiologiquement acceptable.

Par milieu physiologiquement acceptable on entend un milieu compatible avec la peau ou les phanères. Il s'agit en particulier d'un milieu cosmétiquement acceptable. On entend ainsi un milieu ou des excipients bien tolérés par la peau et présentant une odeur et un aspect agréable.

Par produit ou composition cosmétique, on entend une substance ou un mélange destiné à être mis en contact avec les parties superficielles du corps humain ou avec les dents et les muqueuses buccales, en vue, exclusivement ou principalement, de les nettoyer, de les parfumer, d'en modifier l'aspect, de les protéger, de les maintenir en bon état ou de corriger les odeurs corporelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage et/ou produit d'hygiène pour la peau ou les phanères.

La composition selon l'invention peut se présenter sous toutes les formes galéniques normalement utilisées dans le domaine cosmétique, et elle peut être notamment sous forme d'une solution aqueuse ou huileuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

Lorsque la composition utilisée selon l'invention est une émulsion, la proportion de la phase grasse peut aller de 0,5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,1 à 30 % en poids, et de préférence de 0,5 à 20 % en poids par rapport au poids total de la composition.

Comme huiles utilisables dans l'invention, on peut citer les hydrocarbures d'origine minérale (huile minérale) ou synthétique (huile de vaseline, isohexadécane), les huiles d'origine végétale (huile d'amande d'abricot, de pépins de raisins fraction liquide de beurre de karité, huile d'avocat, huile de soja), les huiles d'origine animale (lanoline), les huiles de synthèse (perhydrosqualène, tétraoctanoate de pentaérythrityle), les dérivés siliconés (cyclopentasiloxane, cyclohexasiloxane et polymères de silicone) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille), des beurres, des huiles hydrogénées.

Comme émulsionnants et co-émulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle, les esters de sucrose, les phospoholipides

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur, les chélatants et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'association antioxydante.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges, on peut citer par exemple, les particules de polyamide (Nylon) sous forme sphérique ou sous forme de microfibres ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

La composition utilisée selon l'invention peut en outre contenir d'autres actifs, et notamment au moins un composé choisi parmi : les agents hydratants ; les agents dépigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents tenseurs ; les agents anti-pollution et/ou anti-radicalaire, les filtres solaires, et leurs mélanges.

Comme agents antioxydants, on peut citer notamment le tocophérol et ses esters, en particulier l'acétate de tocophérol ; l'acide ascorbique et ses dérivés, en particulier l'ascorbyl magnésium phosphate, l'ascorbyl glucoside et l'ascorbyl tetraisopalmitate ; l'acide férulique ; la sérine ; l'acide ellagique, la phlorétine, les chélatants, tels que le BHT, le BHA, le N,N'-bis(3,4,5-triméthoxybenzyl) éthylenediamine et ses sels , et leurs mélanges.

En particulier, la composition cosmétique selon l'invention contient en outre au moins un actif choisi parmi la vitamine C , la vitamine E et l'acétate de tocophérol.

Les actifs additionnels pourront être présents dans la composition selon l'invention en une teneur allant de 0,001 % à 20% en poids par rapport au poids total de la composition, de préférence de 0,01% à 10%, encore plus préférentiellement de 0,5% à 5% et de préférence encore de 0,1% à 1% en poids par rapport au poids total de la composition.

L'invention a encore pour objet un procédé de traitement cosmétique comprenant une étape d'application sur la peau ou les phanères de l'association ou des compositions selon l'invention.

Il s'agit en particulier d'un procédé de traitement cosmétique pour prévenir ou diminuer les désordres cutanés induits par un stress oxydatif comprenant au moins une étape consistant à appliquer sur la peau ou les phanères une association d'au moins (a) un polyphénol choisi parmi les monomères et oligomères de proanthocyanidines (OPC), les monomères et oligomères deflavon-3-ols, leurs dérivés et leurs mélanges, les dérivés de polyphénols étant des esters de polyphénols, et (b) un extrait d'épicéa comprenant au moins un lignane choisi parmi hydroxymatairésinol, sécoisolaricirésinol, conidendrine, laricirésinol et liovile, ou une composition cosmétique contenant ladite association.

Le procédé de traitement cosmétique visera ainsi à prévenir ou diminuer les signes cutanés tels qu'un aspect terne du teint, l'hyperpigmentation de la peau ou l'hétérogénéité de sa pigmentation et les taches pigmentaires, les rides et les ridules, l'amincissement de la peau et la perte de fermeté et/ou d'élasticité de la peau.

Le procédé de traitement cosmétique sera également utile pour améliorer l'éclat de la peau et du teint et/ou prévenir ou diminuer l'apparition de cernes autour et/ou sous les yeux, par application d'un extrait d'épicéa, d'une association le contenant ou des compositions tels que définis dans ce qui précède.

Les compositions selon l'invention pourront être appliquées directement sur la peau ou, de façon alternative, sur des supports cosmétiques de type occlusif ou non occlusif, destinés à être appliqués de façon localisée sur la peau, tels que les masques hydrogels, lingettes, tissus, ou biocellulose.

L'application pourra par exemple être quotidienne, pluriquotidienne ou hebdomadaire. Elle pourra être poursuivie pendant plusieurs jours et/ou plusieurs semaines, voire plus longtemps ; elle pourra être continue ou bien par exemple poursuivie pendant 1 à 2 mois puis reprise après une interruption.

L'invention concerne aussi l'utilisation d'une association de
(a) au moins un polyphénol extrait de la vigne choisi parmi les monomères et oligomères de proanthocyanidines (OPC), les monomères et oligomères deflavon-3-ols, leurs dérivés et leurs mélanges, les dérivés étant des esters de polyphénols,
(b) un extrait d'épicéa comprenant au moins un lignane choisi parmi : hydroxymatairésinol, sécoisolaricirésinol, conidendrine, laricirésinol et liovile tels que définis dans le présent texte, pour la préparation d'une composition cosmétique. La composition est adaptée à une application sur la peau ou les phanères. La composition permet avantageusement de lutter contre les signes liés l'action des radicaux libres sur la peau ou les phanères.

L'invention concerne encore un procédé de préparation d'une composition cosmétique comprenant une étape dans laquelle on mélange
(a) au moins un polyphénol choisi parmi les monomères et oligomères de proanthocyanidines (OPC), les monomères et oligomères deflavon-3-ols, leurs dérivés et leurs mélanges, les dérivés étant des esters de polyphénoles, et
(b) un extrait d'épicéa comprenant au moins un lignane choisi parmi : hydroxymatairésinol, sécoisolaricirésinol, conidendrine, laricirésinol et liovile et optionnellement un excipient physiologiquement acceptable.

D'autres caractéristiques et avantages de l'invention sont illustrés dans les exemples qui suivent.

### Exemple 1 : Activité d'un extrait de Picea abies sur les défenses antioxydantes

Une culture confluente de fibroblastes humains à 37°C, en milieu DMEM additionné de 1% de sérum de veau foetal, est traitée par différentes concentrations d'extrait de *Picea abies.*

On teste un extrait de *Picea abies* de composition en lignanes suivantes (% en poids):

| | |
|---|---|
| Lignanes totales : | 6 - 8,4 % |
| Hydroxymatairésinol | 5 - 6 % |
| Sécoisolariciresinol | 0,3 - 0,4 % |
| Conidendrine | 0,2 - 0,4 % |
| Liovile | 0,2 - 0,4 % |
| Laricirésinol | 0,1 - 0,2 % |
| Autres lignanes | 0,2 - 1 % |

- Quantification du GSH dans les lysats cellulaires

Le test GSH/GSSG Glo est basé sur un système de luminescence pour détecter et quantifier le glutathion total (GSH + GSSG) le GSSG et le ratio GSH/GSSG. La détermination est basée sur une réaction de conversion d'une sonde luciférine NT en luciférine par une enzyme glutathion S-transférase couplée à une réaction avec la luciférase. La luminescence détectée est proportionnelle à la quantité de GSH présent.

Dans un cas on mesure la quantité totale de glutathion. Pour cela on convertit l'ensemble du glutathion présent dans le lysat cellulaire en GSH, qui est ensuite mesuré par le test. Dans le cas où seule la forme oxydée GSSG est mesurée, le GSH présent est bloqué par un réactif avant de convertir le GSSG en GSH.

Après incubation, le milieu de culture cellulaire est éliminé et les cellules sont lavées par du PBS, qui est ensuite remplacé par du tampon de lyse.

Les cellules sont lysées pendant 5 mn à température ambiante.

50 µl de «réactif de génération de luciférine» sont ajoutés

Les plaques sont incubées à température ambiante pendant 30 mn.

100 µl / puits de réactif de détection ont été ajoutés

Les plaques ont été équilibrées pendant 15 min à la température ambiante

La luminescence est mesurée avec un appareil TECAN M200 avec les paramètres suivants : temps d'intégration = 1 s, le temps de repos = 1s

Résultat : l'extrait de *Picea abies* à des concentrations allant de 100 à 500µg / ml (0,01% - 0,05%) induit de façon significative la synthèse du GSH cellulaire dans les fibroblastes dermiques humains normaux à partir de 11% jusqu'à 66% par rapport au niveau basal des cellules non traitées.

Ces données ont été confirmées dans une seconde expérience qui a montré qu'un extrait de *Picea abies* riche en hydroxymatairésinol à des concentrations allant 250 - 500 µg / ml (0,025% - 0,05%) est capable d'induire une importante synthèse cellulaire de GSH dans les fibroblastes dermiques humains normaux à partir de 33% jusqu'à 64% par rapport au niveau basal des cellules non traitées.

L'absence de toxicité cellulaire dans ces conditions expérimentales a par ailleurs été vérifiée et permet de conclure que l'effet observé sur la stimulation des défenses n'est pas le résultat d'un effet toxique.
- Quantification de l'enzyme HO-1 dans des lysats cellulaires

Après 24 h de stimulation avec les composés, les cellules ont été détachées, centrifugées 10 mn à 1200 rpm et remises en suspension dans du tampon de lyse. Deux cycles de gel / dégel dans l'azote liquide ont été effectuées puis les fragments cellulaires ont été éliminés par centrifugation. Les lysats cellulaires sont ensuite conservés à -80 °C avant utilisation.

Les concentrations totales de protéines dans des échantillons ont été déterminées en utilisant le kit BCA (Sigma) selon les instructions du fabricant. Pour chaque échantillon, la mesure a été effectuée en triple. La valeur moyenne est rapportée dans les résultats présentés.

La quantité de la HO-1 dans les lysats cellulaires a été mesurée par un test ELISA en utilisant kit ELISA HO-1 / HMOX1 humaine totale, comme suit :
- Des plaques ELISA ont été revêtues d'anticorps de capture à la concentration de 8 µg / ml et incubées à température ambiante pendant une nuit.
- Les plaques ont été lavées 3 fois avec du PBS-0,05% de Tween 20 et bloquées avec du PBS-BSA 1% pendant 1 h à température ambiante.
- Les plaques ont été lavées 3 fois avec du PBS-0,05% Tween 20.
- 100 µl d'échantillons non dilués ont été ajoutés sur des plaques ainsi que des échantillons standards HO-1 à des concentrations allant 0,15 - 10 ng / ml. L'incubation a été effectuée pendant 2 h à température ambiante.
- Après 3 étapes de lavage avec du PBS-Tween 20 0,05%, 100 µl d'anticorps de détection conjugué à HRP (200 ng / ml) a été ajouté pendant 2 h à température ambiante.
- Après 3 étapes de lavage, on ajoute une solution de streptavidine-HRP pour 20 mn à température ambiante.
- Après 3 étapes de lavage, le substrat TMB HRP a été ajouté. Après un temps d'incubation de 15 - 20mn, à température ambiante et à l'abri de la lumière, la réaction a été arrêtée par addition de H2SO4 2N.
- La lecture de densité optique (DO) a été effectuée avec le lecteur de microplaques Tecan Infini à 450 nm avec correction à 570 nm.

Les résultats ont été analysés avec le logiciel Magellan.

Les concentrations d'HO-1 ont été normalisées à une concentration totale de protéines pour chaque échantillon.

L'absence de toxicité cellulaire dans ces conditions expérimentales a par ailleurs été vérifiée et permet de conclure que l'effet observé sur la stimulation des défenses n'est pas le résultat d'un effet toxique.

Résultats: concentrations de HO-1 (pg/µg de protéines totales)

| | | Moyenne des 3 échantillons | S.D. | p-value | augmentation (%) |
|---|---|---|---|---|---|
| Non traité | 0 | 11,75 | 0,54 | | |
| Extrait de *Picea abies* à 8%(µg/ml) | 100 | 12,01 | 1,53 | 0,7973 | 2,19 |
| | 500 | 36,74 | 2,53 | <0,0001 | 212,63 |

### Exemple 2 : Activité d'une association d'un extrait de Picea abies et de pépin de raisin sur les défenses antioxydantes

Une culture confluente de fibroblastes humains à 37°C, en milieu DMEM additionné de 1% de sérum de veau foetal, est traitée par différentes concentrations d'extrait de *Picea abies* et d'extrait de pépin de raisin, selon le même protocole que dans l'exemple 1.

Les extraits de pépin de raisin ont une teneur en proanthocyanidines par rapport aux polyphénols totaux suivante :

| | |
|---|---|
| Polyphenols totaux | 100% |
| Catéchine + épicatéchine | 22,6% |
| Dimères de proanthocyanidine | 25,1% |

Les concentrations de HO-1 dans les lysats cellulaires ont été normalisées par rapport à la concentration totale de protéine dans chaque échantillon. Les résultats sont présentés dans le tableau suivant:

| | | Concentrations de HO-1 (pg/µg de protéines totales) | | | |
|---|---|---|---|---|---|
| | | Moyenne des 3 mesures | **S.D** | **p value (t-test)** | **augmentation (%)** |
| Non traité | 0 | 18,54 | 2,40 | | 0,00 |
| Extrait *Picea abies à 8%* | 500 | 24,84 | 3,98 | 0,079 | 33,94 |
| (µg/ml) | | | | | |
| Extrait de pépin de raisin | 14,5 | 31,12 | 3,10 | 0,0052 | 67,83 |
| (µg/ml) | | | | | |
| Extrait *Picea abies* à 8%+ Extrait de pépin de raisin (µg/ml) | 500 + 14,5 | 65,90 | 9,36 | 0,0011 | 255,43 |

L'absence de toxicité cellulaire dans ces conditions expérimentales a par ailleurs été vérifiée et permet de conclure que l'effet observé sur la stimulation des défenses n'est pas le résultat d'un effet toxique.

Le pourcentage d'augmentation de l'activité pour un effet additif aurait été de 34% + 68% soit 102%. L'activité mesurée pour l'association d'extrait de *Picea abies* et d'extrait de pépin de raisin est supérieure de 76% à cette somme théorique.

Cela démontre qu'un extrait de pépins de raisin enrichi en proanthocyanydines et un extrait de *Picea abies* riche en hydroxymatairésinol sont capables d'induire de manière significative la production de l'enzyme anti-oxydante HO-1 dans les fibroblastes dermiques humains normaux d'une manière synergique. En effet, l'activité mesurée pour l'association est supérieure à la somme des activités mesurées pour chaque composé individuellement.

Les résultats sont résumés dans le tableau ci-dessous :

| | | Défense antioxydante enzymatique : Augmentation de HO-1 (%) |
|---|---|---|
| Extrait de *Picea abies* à 8% | 500 µg/ml | 34% |
| Extrait de pépin de raisin | 14,5 µg/ml | 68% |
| Extrait de *Picea abies* à 8% + Extrait de pépin de raisin (activité théorique pour un effet additif) | 500 µg/ml + 14,5 µg/ml | 102% |
| Extrait de *Picea abies* à 8% + Extrait de pépin de raisin | 500 µg/ml + 14,5 µg/ml | 255% |

### Exemple 3 : Compositions selon l'invention

### Lotion

| Nom Commun | % |
|---|---|
| Eau | QSP 100 |
| Glycérine | 3,00 |
| Melange de methylpropane diol, caprylyl glycol, phenyl propanol | 0,50 |
| Extrait de *Picea abies* à 8% | 0,20 |
| Polyphenols de pépins de raisin | 0,25 |
| Alcool | 5,00 |

On mélange les différents constituants de la composition pour obtenir une lotion. Celle-ci sera appliquée le matin et/ou le soir sur l'ensemble du visage et du cou.

### Gel Crème

| Nom Commun | % |
|---|---|
| Eau | QSP 100 |
| Butylène glycol | 3,00 |
| C10/30 alkyacrylates crosspolymer | 0,50 |
| Gomme xanthane | 0,30 |
| Coco-caprylate | 5,00 |
| Huile de pépins de raisin | 3,00 |
| Extrait de *Picea abies* à 8% | 1,00 |
| Dérivés de Polyphenols de vigne | 0,60 |
| Sorbate de potassium | 0,20 |
| Parfum | 0,30 |

Le gel crème sera appliqué matin et/ou soir sur le visage, le cou et les mains.

### Emulsion

| Nom Commun | % |
|---|---|
| Eau | QSP 100 |
| C10/30 alkyacrylates crosspolymer | 0,30 |
| Acide phytique | 0,05 |
| Mélangé de methylpropane diol, caprylyl glycol, phenyl propanol | 2,00 |
| Dicaprylyl ether | 20,00 |
| Mélange de cetearyl alcohol et PEG-20 stéarate | 3,00 |
| Extrait de *Picea abies* à 8% | 1,00 |
| Dérivés de Polyphenols de vigne | 0,3 |

La composition sera appliquée matin et/ou soir sur le visage et le cou

## Revendications

1. Composition cosmétique contenant au moins une association de
(a) au moins un polyphénol extrait de la vigne choisi parmi les monomères et oligomères de proanthocyanidines (OPC), les monomères et oligomères de flavan-3-ols, leurs dérivés et leurs mélanges, les dérivés de polyphénols étant des esters de polyphénols,
(b) un extrait d'épicéa comprenant au moins un lignane choisi parmi : hydroxymatairésinol, sécoisolaricirésinol, conidendrine, laricirésinol et liovile.

2. Composition cosmétique selon la revendication 1, **caractérisée en ce que** le ou les polyphénol(s) mentionnés en (a) sont extraits de la vigne et comprennent des OPC extraits de pépin de raisin.

3. Composition cosmétique selon l'une au moins des revendications 1 ou 2, **caractérisée en ce que** les polyphénols ou les dérivés de polyphénols (a) sont présents à une concentration de 0,01 à 10% en poids par rapport au poids total de la composition.

4. Composition cosmétique selon l'une au moins des revendications 1 à 3, **caractérisée en ce que** l'extrait d'épicéa est un extrait de bois d'épicéa et est présent à une concentration de 0,01 à 10% en poids par rapport au poids total de la composition.

5. Composition cosmétique selon l'une au moins des revendications 1 à 4, **caractérisée en ce qu'**elle contient au moins
(a) un extrait de pépin de raisin contenant des polyphénols, stabilisé par estérification, l'extrait étant présent à une concentration de 0,01 à 10% en poids
(b) un extrait d'épicéa à une concentration de 0,01 à 10% en poids.

6. Composition cosmétique selon l'une au moins des revendications 1 à 5, **caractérisée en ce que** le rapport massique des concentrations entre les polyphénols extraits de vigne ou leurs dérivés et l'extrait d'épicéa est compris entre 0,1 et 5 exprimé en polyphénols/ lignanes d'épicéa.

7. Composition cosmétique selon l'une au moins des revendications 1 à 6, **caractérisée en ce qu'**elle contient en outre au moins un actif choisi parmi les agents antioxydants, les agents hydratants, les agents desquamants, les agents améliorant la fonction barrière, les agents dépigmentants, les agents favorisant la microcirculation cutanée.

8. Composition cosmétique selon l'une au moins des revendications 1 à 7, **caractérisée en ce qu'**elle contient au moins un actif choisi parmi la vitamine C, la vitamine E et l'acétate de tocophérol.

9. Utilisation cosmétique d'une association de (a) au moins un polyphénol extrait de la vigne choisi parmi les monomères et oligomères de proanthocyanidines (OPC), les monomères et oligomères de flavan-3-ols, leurs dérivés et leurs mélanges, les dérivés de polyphénols étant des esters de polyphénols, et (b) un extrait d'épicéa comprenant au moins un lignane choisi parmi hydroxymatairésinol, sécoisolaricirésinol, conidendrine, laricirésinol et liovile, ou d'une composition cosmétique la contenant, tels que définis dans l'une des revendications 1 à 8 pour lutter contre les signes du vieillissement de la peau ou des phanères ou contre les signes cutanés liés à la pollution.

10. Utilisation cosmétique d'une association de (a) au moins un polyphénol extrait de la vigne choisi parmi les monomères et oligomères de proanthocyanidines (OPC), les monomères et oligomères de flavan-3-ols, leurs dérivés et leurs mélanges, les dérivés de polyphénols étant des esters de polyphénols, et (b) un extrait d'épicéa comprenant au moins un lignane choisi parmi hydroxymatairésinol, sécoisolaricirésinol, conidendrine, laricirésinol et liovile, ou d'une composition cosmétique la contenant, tels que définis dans l'une des revendications 1 à 8, pour améliorer l'éclat de la peau et/ou diminuer l'apparition de cernes autour des yeux.

11. Utilisation cosmétique d'une association selon l'une au moins des revendications 9 ou 10, **caractérisée en ce que** l'association ou la composition la contenant est utilisée comme agent pour lutter contre un désordre choisi parmi les rides et les ridules, le teint terne, les taches pigmentaires, la perte de fermeté de la peau et les cernes sombres sous les yeux.

12. Procédé de traitement cosmétique pour prévenir ou diminuer les désordres cutanés induits par un stress oxydatif comprenant au moins une étape consistant à appliquer sur la peau ou le cuir chevelu une association d'au moins (a) de polyphénols choisis parmi les monomères et oligomères de proanthocyanidines (OPC), les monomères et oligomères de flavan-3-ols, leurs dérivés de polyphénols qui sont des esters de polyphénols et leurs mélanges, et (b) un extrait d'épicéa comprenant au moins un lignane choisi parmi hydroxymatairésinol, sécoisolaricirésinol, conidendrine, laricirésinol et liovile, ou une composition cosmétique contenant ladite association.

## Patentansprüche

1. Kosmetische Zusammensetzung, die mindestens eine Kombination enthält aus
a) mindestens einem aus Weinreben extrahierten Polyphenol, das ausgewählt ist aus den Monomeren und Oligomeren von Proanthocyanidinen (OPC), den Monomeren und Oligomeren von Flavan-3-olen, ihren Derivaten und ihren Gemischen, wobei es sich bei den Polyphenolderivaten um Polyphenolester handelt,
b) einem Fichtenextrakt, das mindestens ein Lignan umfasst, das ausgewählt ist aus: Hydroxymatairesinol, Secoisolariciresinol, Conidendrin, Lariciresinol und Liovil.

2. Kosmetische Zusammensetzung nach Anspruch 1, **dadurch gekennzeichnet, dass** das oder die in (a) erwähnten Polyphenol(e) aus Weinreben extrahiert sind und OPC umfassen, die aus Traubenkernen extrahiert sind.

3. Kosmetische Zusammensetzung nach mindestens einem der Ansprüche 1 oder 2, **dadurch gekennzeichnet, dass** die Polyphenole oder Polyphenolderivate (a) in einer Konzentration von 0,01 bis 10 Gew.-%, bezogen auf das Gesamtgewicht der Zusammensetzung, vorhanden sind.

4. Kosmetische Zusammensetzung nach mindestens einem der Ansprüche 1 bis 3, **dadurch gekennzeichnet, dass** es sich bei dem Fichtenextrakt um ein Fichtenholzextrakt handelt und es in einer Konzentration von 0,01 bis 10 Gew.-%, bezogen auf das Gesamtgewicht der Zusammensetzung, vorhanden ist.

5. Kosmetische Zusammensetzung nach mindestens einem der Ansprüche 1 bis 4, **dadurch gekennzeichnet, dass** sie mindestens
a) einen polyphenolhaltigen Traubenkernextrakt, der durch Veresterung stabilisiert ist, wobei der Extrakt in einer Konzentration von 0,01 bis 10 Gew.-% vorliegt
b) einen Fichtenextrakt in einer Konzentration von 0,01 bis 10 Gew.-% enthält.

6. Kosmetische Zusammensetzung nach mindestens einem der Ansprüche 1 bis 5, **dadurch gekennzeichnet, dass** das Massenverhältnis der Konzentrationen zwischen den aus Weinreben extrahierten Polyphenolen oder deren Derivaten und dem Fichtenextrakt zwischen 0,1 und 5 liegt, ausgedrückt als Fichtenpolyphenole/-lignane.

7. Kosmetische Zusammensetzung nach mindestens einem der Ansprüche 1 bis 6, **dadurch gekennzeichnet, dass** sie außerdem mindestens einen Wirkstoff enthält, der ausgewählt ist aus Antioxidationsmitteln, feuchtigkeitsspendenden Mitteln, schuppenlösenden Mitteln, Mitteln zur Verbesserung der Barrierefunktion, depigmentierenden Mitteln und Mitteln zur Förderung der Mikrozirkulation der Haut.

8. Kosmetische Zusammensetzung nach mindestens einem der Ansprüche 1 bis 7, **dadurch gekennzeichnet, dass** sie mindestens einen Wirkstoff enthält, der ausgewählt ist aus Vitamin C, Vitamin E und Tocopherolacetat.

9. Kosmetische Verwendung einer Kombination aus (a) mindestens einem aus Weinreben extrahierten Polyphenol, das ausgewählt ist aus den Monomeren und Oligomeren von Proanthocyanidinen (OPC), den Monomeren und Oligomeren von Flavan-3-olen, ihren Derivaten und ihren Gemischen, wobei es sich bei den Polyphenolderivaten um Polyphenolester handelt, und (b) einem Fichtenextrakt, der mindestens ein Lignan enthält, das ausgewählt ist aus Hydroxymatairesinol, Secoisolariciresinol, Conidendrin, Lariciresinol und Liovil, oder einer kosmetischen Zusammensetzung, die diese enthält, wie in einem der Ansprüche 1 bis 8 definiert, zum Bekämpfen von Alterungserscheinungen der Haut oder der Hautanhangsgebilde oder von Hauterscheinungen, die mit der Umweltverschmutzung in Zusammenhang stehen.

10. Kosmetische Verwendung einer Kombination aus (a) mindestens einem aus Weinreben extrahierten Polyphenol, das ausgewählt ist aus den Monomeren und Oligomeren von Proanthocyanidinen (OPC), den Monomeren und Oligomeren von Flavan-3-olen, ihren Derivaten und ihren Gemischen, wobei es sich bei den Polyphenolderivaten um Polyphenolester handelt, und (b) einem Fichtenextrakt, der mindestens ein Lignan enthält, das ausgewählt ist aus Hydroxymatairesinol, Secoisolariciresinol, Conidendrin, Lariciresinol und Liovil, oder einer kosmetischen Zusammensetzung, die diese enthält, wie in einem der Ansprüche 1 bis 8 definiert, um den Glanz der Haut zu verbessern und/oder um das Auftreten von dunklen Ringen um die Augen zu verringern.

11. Kosmetische Verwendung einer Kombination nach mindestens einem der Ansprüche 9 oder 10, **dadurch gekennzeichnet, dass** die Kombination oder die sie enthaltende Zusammensetzung als Mittel zur Bekämpfung einer Störung verwendet wird, die ausgewählt ist aus Falten und Fältchen, fahlem Teint, Pigmentflecken, Verlust der Hautfestigkeit und dunklen Ringen unter den Augen.

12. Verfahren zur kosmetischen Behandlung zur Vorbeugung oder Verringerung von Hautstörungen, die durch oxidativen Stress induziert werden, mindestens einen Schritt umfassend, bei dem auf die Haut oder die Kopfhaut eine Kombination aufgetragen wird aus mindestens (a) Polyphenolen, die ausgewählt sind aus den Monomeren und Oligomeren von Proanthocyanidinen (OPC), Monomeren und Oligomeren von Flavan-3-olen, ihren Polyphenolderivaten, bei denen es sich um Polyphenolester und Gemische davon handelt, und (b) einem Fichtenextrakt, der mindestens ein Lignan enthält, das ausgewählt ist aus Hydroxymatairesinol, Secoisolariciresinol, Conidendrin, Lariciresinol und Liovil, oder eine kosmetische Zusammensetzung, die diese Kombination enthält.

## Claims

1. A cosmetic composition containing at least one combination of
(a) at least one polyphenol extracted from the vine chosen from monomers and oligomers of proanthocyanidins (OPCs), monomers and oligomers of flavan-3-ols, derivatives thereof and mixtures thereof, the polyphenol derivatives being polyphenol esters,
(b) a spruce extract comprising at least one lignan chosen from: hydroxymatairesinol, secoisolariciresinol, conidendrin, lariciresinol and liovile.

2. The cosmetic composition according to claim 1, **characterized in that** the polyphenol(s) mentioned in (a) are extracted from the vine and comprise OPCs extracted from grape seed.

3. The cosmetic composition according to at least one of claims 1 or 2, **characterized in that** the polyphenols or polyphenol derivatives (a) are present at a concentration of 0.01 to 10% by weight relative to the total weight of the composition.

4. The cosmetic composition according to at least one of claims 1 to 3, **characterized in that** the spruce extract is a spruce wood extract and is present at a concentration of 0.01 to 10% by weight relative to the total weight of the composition.

5. The cosmetic composition according to at least one of claims 1 to 4, **characterized in that** it contains at least
(a) a grape seed extract containing polyphenols, stabilized by esterification, the extract being present at a concentration of 0.01 to 10% by weight
(b) a spruce extract at a concentration of 0.01 to 10% by weight.

6. The cosmetic composition according to at least one of claims 1 to 5, **characterized in that** the mass ratio of the concentrations between the polyphenols extracted from vine or derivatives thereof and the spruce extract is between 0.1 and 5 expressed in polyphenols/spruce lignans.

7. The cosmetic composition according to at least one of claims 1 to 6, **characterized in that** it additionally contains at least one active ingredient chosen from antioxidant agents, moisturizing agents, desquamating agents, agents improving the barrier function, depigmenting agents, agents promoting skin microcirculation.

8. The cosmetic composition according to at least one of claims 1 to 7, **characterized in that** it contains at least one active ingredient chosen from vitamin C, vitamin E and tocopherol acetate.

9. A cosmetic use of a combination of (a) at least one polyphenol extracted from the vine chosen from monomers and oligomers of proanthocyanidins (OPCs), monomers and oligomers of flavan-3-ols, derivatives thereof and mixtures thereof, the polyphenol derivatives being polyphenol esters, and (b) a spruce extract comprising at least one lignan chosen from hydroxymatairesinol, secoisolariciresinol, conidendrin, lariciresinol and liovile, or of a cosmetic composition containing it, as defined in any of claims 1 to 8 for combating the signs of aging of the skin or skin appendages or the cutaneous signs related to pollution.

10. A cosmetic use of a combination of (a) at least one polyphenol extracted from the vine chosen from monomers and oligomers of proanthocyanidins (OPCs), monomers and oligomers of flavan-3-ols, derivatives thereof and mixtures thereof, the polyphenol derivatives being polyphenol esters, and (b) a spruce extract comprising at least one lignan chosen from hydroxymatairesinol, secoisolariciresinol, conidendrin, lariciresinol and liovile, or of a cosmetic composition containing it, as defined in any of claims 1 to 8, for improving the radiance of the skin and/or reducing the appearance of dark circles around the eyes.

11. The cosmetic use of a combination according to at least one of claims 9 or 10, **characterized in that** the combination or the composition containing it is used as an agent for combating a disorder chosen from wrinkles and fine lines, dull complexion, pigmentation spots, loss of skin firmness and dark circles under the eyes.

12. A cosmetic treatment method for preventing or reducing cutaneous disorders induced by oxidative stress comprising at least one step consisting in applying to the skin or scalp a combination of at least (a) polyphenols chosen from monomers and oligomers of proanthocyanidins (OPCs), monomers and oligomers of flavan-3-ols, polyphenol derivatives thereof which are polyphenol esters and mixtures thereof, and (b) a spruce extract comprising at least one lignan chosen from hydroxymatairesinol, secoisolariciresinol, conidendrin, lariciresinol and liovile, or a cosmetic composition containing said combination.
